# EUROPEAN PATENT SPECIFICATION

(11) **EP 2 649 991 B1**
(45) Date of publication and mention of the grant of the patent: **24.02.2016**
(21) Application number: 12164045.2
(22) Date of filing: 13.04.2012
(51) Int. Cl.: A61K 31/045, A61K 31/575, A61K 31/7048, A61K 36/47, A61P 1/00

(54) **Extracts and isolated compounds from Euphorbia granulata Forrsk. for treatment of ulcerative colitis**
Extrakte und isolierte Verbindungen aus Euphorbia granulata Forrsk. zur Behandlung von Colitis ulcerosa
Extraits et composés isolés à partir de l'euphorbia granulata forrsk pour le traitement de la rectocolite hémorragique

(43) Date of publication of application: 16.10.2013
(73) Proprietor: King Saud University, 11421 Riyadh (SA)
(72) Inventor: Awaad, Amani Shafeek, 11333 Riyadh (SA); Al-Jaber, Nabila, 11333 Riyadh (SA); Al-Meteary, Hanoof Saoud, 11333 Riyadh (SA); El-Desouky Zain, Mohamed, 11333 Riyadh (SA); El-Meligy, Reham Moustafa, 11333 Riyadh (SA)
(74) Representative: Goddar, Heinz J.

(56) References cited:
- US-B1- 7 211 567
- ZAKARIA SERAG ET AL: "Phytotherapy for ulcerative colitis-Results of a pilot study using beta-sitosterol as MEBO", GASTROENTEROLOGY, ELSEVIER, PHILADELPHIA, PA, vol. 116, no. 4 PART 2, 1 April 1999 (1999-04-01), page A850, XP008101940, ISSN: 0016-5085
- AHMAD V U ET AL: "CHEMICAL CONSTITUENTS OF EUPHORBIA-GRANULATA", FITOTERAPIA, IDB HOLDING, MILAN, IT, vol. 57, no. 4, 1 January 1986 (1986-01-01), page 280, XP009161373, ISSN: 0367-326X
- KAWASHTY, S. A. ET AL: "Chemosystematic studies of Euphorbia cuneta and Euphorbia", BULLETIN OF THE NATIONAL RESEARCH CENTRE (EGYPT) , 21(4), 411-417 CODEN: BNRCET, vol. 21, no. 4, 1996, pages 411-417, XP9161427,
- ATIQUR RAHMAN M ET AL: "Medicinal plant diversity in the flora of Saudi Arabia 1: a report on seven plant families", FITOTERAPIA, IDB HOLDING, MILAN, IT, vol. 75, 1 January 2004 (2004-01-01), pages 149-161, XP007912093, ISSN: 0367-326X, DOI: 10.1016/J.FITOTE.2003.12.012
- RIZK A M ET AL: "Constituents of plant growing in Qatar. II. Investigation of Euphorbia granulata", DIE PHARMAZIE, GOVI VERLAG PHARMAZEUTISCHER VERLAG GMBH, ESCHBORN, DE, vol. 37, no. 10, 1 January 1982 (1982-01-01), pages 737-738, XP001526189, ISSN: 0031-7144
- SEYYEDNEJAD S M ET AL: "A review on native medicinal plants in Khuzestan, Iran with antibacterial properties", INTERNATIONAL JOURNAL OF PHARMACOLOGY 2010 ASIAN NETWORK FOR SCIENTIFIC INFORMATION PAK, vol. 6, no. 5, 2010, pages 551-560, XP002680678, ISSN: 1811-7775
- AHMAD ET AL: "Antifungal and antispasmodic activities of the extracts of Euphorbia granulata", JOURNAL OF MEDICINAL PLANTS RESEARCH, vol. 6, no. 1, 9 January 2012 (2012-01-09) , pages 19-23, XP002680679,
- HEAD KATHLEEN A ET AL: "Inflammatory bowel disease Part 1: ulcerative colitis--pathophysiology and conventional and alternative treatment options", ALTERNATIVE MEDICINE REVIEW, THORNE RESEARCH INC., SANDPOINT, US, vol. 8, no. 3, 1 August 2003 (2003-08-01), pages 247-283, XP002349422, ISSN: 1089-5159

## Description

The present invention relates to extracts from *Euphorbia granulata* Forssk., compounds isolated from *Euphorbia granulata* Forrsk. and their use for the treatment of ulcerative colitis.

The use of plants or plant materials as therapeutic agents is well known for a long time. However, in the most recent decades, synthesized drugs were preferably used for the treatment of diseases which, however, have a variety of negative side effects. To minimize or avoid the side effects of synthesized drugs, man started to return back to nature in order to discover alternative, natural curing agents. *Euphorbiaceae* is one of the largest plant families (321 genera and 7950 species). Most of its species contain a milky or colored latex. This latex is poisonous in some of the species and many of them further contain irritant and pesticidal substances. The Genus *Euphorbia* constitutes the largest number of this family. It comprises 700 species of trees, shrubs or herbs with acrid milky juice.

This genus is of great importance due to its various phytochemical constituents as phenolic compounds, terpenoids or tannins. In addition, it was well known because of its variable medical uses, such as acetylcholine-like action with muscarinic and nicotinic activities on isolated ileum of rabbit, spasmolytic, diuretic, increase of capillary strength, antileukemic, antiinflammatory analgesic and decreased the release of prostaglandin.

Dexamethasone is a drug commonly used for the treatment of ulcerative colitis. However, when using dexamethasone in medical applications, several side effects such as nausea, weight gain and fatigue were observed.

Fedorak et al., Gastroenterology, 1995, 108(6), 1688-1699 relates to the use of dexamethasone for healing of colitis in rats.

Kenyon et al., Aliment. Pharmacol. Ther. 1997, 11(1), 205-213, discloses colonic delivery of dexamethasone in the treatment of ulcerative colitis.

Sood et al., J. Clin. Gastroenterol. 2002, 35(4), 328-331 relates to a prospective, open-label trial assessing dexamethasone pulse therapy in moderate to severe ulcerative colitis.

It is an object of the present invention to provide novel and efficient agents for the treatment of ulcerative colitis which overcome the drawbacks of the prior art and which, particularly, can be obtained from natural materials.

This object is achieved by an extract from a plant material of *Euphorbia granulata* Forssk. for use in the treatment of ulcerative colitis.

Preferably, the extract is an alcoholic extract, more preferably is an ethanolic extract.

Most preferred, the plant material comprises the aerial parts of *Euphorbia granulata* Forssk.

The object is further achieved by a compound selected from heptacosan-1-ol, kaempferol-3-glycoside and/or kaempferol-3-galactoside for use in the treatment of ulcerative colitis.

The object is also achieved by a method for isolating heptacosan-1-ol, and/or kaempferol-3-galactosid from *Euphorbia granulata* Forssk., comprising the steps:
a) extracting a plant material of *Euphorbia granulata* Forssk. to obtain an extract;
b) concentrating the extract to obtain a concentrate;
c) chromatographing the concentrate into fractions; and
d) isolating fractionated compounds.

It is preferred that the plant material comprises the aerial parts of *Euphorbia granulata* Forssk.

Furthermore, it is preferred that extracting is carried out by using at least one organic solvent.

In a preferred embodiment, the organic solvent is selected from the group consisting of petroleum ether, heptane, hexane, ethanol, isopropanol, methanol and mixtures thereof, preferably from the group consisting of petroleum ether and ethanol.

Most preferred, the chromatographing is carried out by means of thin layer chromatography (TLC) and/or column chromatography.

Preferably, the eluent used for chromatographing is petroleum ether, heptane, ethanol, methanol, benzene, diethyl ether, chloroform, dichloromethane, water and/or mixtures thereof.

It is further preferred that the stationary phase used for chromatographing is silica gel or aluminum oxide.

Finally, the object is achieved by a pharmaceutical composition comprising an extract according to claim 1 and/or at least one of the compounds according to claim 4.

Preferably, the composition is formulated for oral administration.

Surprisingly, it was found that extracts from *Euphorbia granulata* Forssk. as well as compounds isolated thereof solve the problem by providing natural agents for the treatment of ulcerative colitis which exhibit similar or even higher anti-ulcerative colitis activity compared to drugs known from the prior art. Further, it was found that the extract and compounds can be used for the treatment of ulcerative colitis with no side effects on the liver and kidney functions.

Further, it was surprisingly found that several compounds having anti-ulcerative colitis activity can be isolated from *Euphorbia granulata* Forssk. by the inventive method in an easy and efficient way.

"Extracting" in terms of the present invention, for preparing the inventive extract or in the first step of the inventive method, can comprise only one or more than one consecutive extraction steps. In the latter case, different solvents or solvent mixtures, in particular solvents or solvent mixtures having a significantly different polarity, can be used in the varioius steps.

In the same way, "chromatographing" in terms of the present invention can comprise only one chromatographic separation as well as more than one consecutive chromatographic separation steps. Particularly, different eluents and/or stationary phases can be used in each chromatography step. Further, in case that two or more chromatographic separation steps are carried out, each step can be a different chromatographic method, for example in the first step a mixture (concentrate) is separated by column chromatography and single fraction obtained in the first step is subsequently separated by thin layer chromatography.

The term "pharmaceutical composition", as used herein, is intended to comprise at least one pharmaceutically active extract of the present invention and/or at least one of the isolated compound of the present invention and/or corresponding salts thereof.

The pharmaceutical composition can be, for example, in a liquid form, e.g. a solution, sirup, elixir, emulsion and suspension, or in a solid form, e.g. a capsule, caplet, tablet, pill, powder and suppository. Granules, semi-solid forms and gel caps are also considered. In case that the pharmaceutical composition is a liquid or a powder, dosage unit optionally is to be measured, e.g. in the dosage unit of a teaspoon. In addition to one of the extracts or the isolated compounds, the pharmaceutical composition can comprise, for example, flavoring agents, sweeteners, dyes, stabilizers, diluents, suspending agents, granulating agents, lubricants, binders and disintegrating agents. A tablet, for example, can be coated. All of the formulations mentioned can be intended for immediate-release, timed-release and sustained release.

All components of the pharmaceutical composition have to be pharmaceutically acceptable. The term "pharmaceutically acceptable" means at least non-toxic. The therapeutically active compound should preferably be present in the above-mentioned pharmaceutical composition in a concentration of about 0.1 to 99.5% by weight, preferably of about 0.5 to 95% by weight of the total mixture.

The above-mentioned pharmaceutical composition can further contain other pharmaceutical active compounds in addition to the active extracts and/or compounds according to the invention.

Additional features and advantages of the present invention will become apparent in the following detailed description on the basis of the examples.

### Examples

### Material and methods

**Plant materials:** The aerial parts of *Euphorbia granulata* Forssk. (Euphorpiaceae) were collected during flowering stage in 2010 from Desert of Saudi Arabia. The sample was identified, especially by comparison with the published plant description (El-Gohary, 2004). A voucher specimen has been deposited in the herbarium of Chemistry department, faculty of Sciences, King Saud University. The plant material was air-dried in shade, reduced to fine powder, packed in tightly closed containers and stored for phytochemical and biological studies.

***Apparatus:*** Melting points were determined on a Kofler hot-stage apparatus and are uncorrected.

Mass spectra (Electrospray negative ion) were taken from samples dissolved in acetonitrile with a Micromass Quattro spectrometer.

¹H- and ¹³C-NMR spectra, using external electronic referencing through the deuterium resonance frequency of the solvent, were determined at 600.17 or 150.91 MHz respectively with a JEOL ECA600 NMR spectrometer fitted with an auto-tune 5mm X/H probe. Carbon atom types were established in the ¹³CNMR spectrum by employing a combination of broad-band proton-decoupled and DEPT (90 and 135) experiments. [¹*J*_{C-H}], [²*J*_{C-H}] and [³*J*_{C-H}] ¹H-¹³C correlations were determined by using HMQC and HMBC pulse sequences. ¹H-¹H correlations were determined by double quantum filtered COSY.

Pye Unicam pu 8800 spectrophotometer was used for UV spectral analysis.

Amino acid analysis was carried out using the amino acid analyzer (Eppendorf-LC 3000).

IR spectra were taken with a Shimadzu - IR-435 infrared spectrophotometer.

***Phytochemical screening:*** Powdered samples of the aerial parts of *E. granulata* were subjected to preliminary phytochemical screening according to the published methods (Awaad, 2009).

### Example 1

### Extraction and isolation:

### Extraction of plant material:

One kilogram of the air dried powder of *E. granulata* (aerial parts) was extracted by percolation using petroleum ether at 60- 80°C for two days and filtered off. This process was repeated for three times. The combined petroleum ether extracts were concentrated under reduced pressure at a temperature not exceeding 25°C to yield a dry extract of 120 g (**E1**).

The plant powder kept after the extraction with petroleum ether was further extracted with ethanol at room temperature for two days and filtered. The residue was re-percolated again. The combined ethanol extracts were concentrated under reduced pressure at a temperature not exceeding 35°C to yield a dry extract of 280 g (**E2**).

### Isolation:

**(A)** 10 g of **E1** extract were dissolved in chloroform and mixed with alumina for column chromatography. The solvent was evaporated on steam bath with continuous titration to form a free flowing powder. The obtained powder was applied on the top of glass column (150 X 2.5 cm) packed with alumina (300 g).

Elution was carried out gradually using benzene-ethyl acetate (30 dps/min.). Two hundred fraction were collected (50 ml each) and reduced to three sub-fractions, after chromatographic examination using two solvent systems (benzene-ethyl acetate 86:14; benzene - ethyl acetate 80:20).

**Sub-fractions I** (1.20g) was cleaned and tested for its anti-ulcerative colitis activity. Since no significant activity was observed, sub-fraction I was discarded.

**Sub-fraction II** (1.05 g) contained three fractions having R_{f} = 0.60, 0.48 and 0.45. One of them was the major and others were minor. For the isolation of the major spot column chromatography (1 X 50 cm) packed with silica gel (30 g) and benzene-diethylether 1:1 v/v as eluent was used. Fifteen fractions were collected and reduced to two sub-fractions, each containing semi purified, spots. For final purification preparative TLC plates were used and developed on benzene-diethylether 8:2 v/v. The band corresponding to the major compound was collected and extracted by chloroform. The solvent was evaporated using high pressure at low temperature to obtain heptacosan-1-ol.

**Sub-fraction III** (1.40g) was collected and evaporated to dryness. This fraction contained one spot with R_{f}= 0.33. Recrystallization from methanol yielded β-sitosterol.
**(B) E2** extract (12 g) was dissolved in chloroform and mixed with silica gel G for column chromatography. The solvent was evaporated on water bath with continuous titration to form a free flowing dry powder. The powdered homogenate was applied on to the top of a glass column (5 cm × 1.5 m) packed with 360 g silica gel G. Elution was gradually carried out using benzene-ethyl acetate.

Fractions (100 ml each) were collected (137 fraction) and chromatographically screened on silica gel G TLC plates using ethyl acetate- methanol- water 70:5:4. Visualization was achieved under UV before and after spraying with AlCl₃. Similar fractions were collected together to produce two sub- fractions.

**Sub-fraction I** was purified and tested for anti-ulcerative colitis activity. After obtaining no significant activity, the respective sub-fraction was discarded.

**Sub-fraction II** contained four compounds with a total weight of 4 g. It was dissolved in 5 ml methanol, mixed with silica gel G and dried to form a fluently powder. The dry powder was applied on the top of glass column (1.5×50 cm) packed with 80 g silica gel. Elution was carried out using chloroform -methanol 98:2 v/v. The collected 25 fractions were dried under reduced pressure at 35 C and reduced to two main sub-fraction.

Each sub-fraction was purified using preparative TLC on silica gel G with chloroform - methanol 98:2 v/v. The bands of the developed chromatograms corresponding to the major compounds were scraped and extracted with methanol. The eluates were concentrated to dryness, purified using a column (1×20 cm) packed with 20 g silica gel G. Elution was proceeded gradually using chloroform - methanol. The individually collected sub-fractions were dried under reduced pressure at about 35°C to obtain kaempferol-3-O-β-D-glucoside and kaempferol-3-galactoside.

### Heptacosan-1-ol

This compound was obtained as whitish crystal residue (26.3 mg) with R_{f} = 0.33 in benzene-ethyl acetate 86:14. **¹H-NMR** : (Chloroform-D3) δ 3.62 ppm (2H q, *J*= 5.52 Hz, H- 2) this proton near to -OH group, δ 1.55 ppm (2H q , *J*= 7.38 Hz, H-3) this proton between two CH₂ , δ 1.28 ppm (48H m, (CH₂)₂₄ H- 3→26), δ 0.86 ppm (3H t, *J*=7.08 Hz, H-28) **¹³C-NMR:** (Chloroform-D3) showed 13 carbon 9 carbon similar. **HMQC:** (chloroform-D3): Correlation between δ 0.86 ppm for H-25 and δ 14.27 ppm for C-25. Correlation between δ 1.27 ppm for H-3 and δ 22.83 ppm for C-3 Correlation between δ 1.27 ppm for H-2 and δ 25.86 ppm for C-2. Correlation between δ 1.27 ppm for H-4--24 and δ 29.81-29.50 ppm for C-4-24. Correlation between δ 1.55 ppm for H-27 and δ 32.06 ppm for C-27. Correlation between δ 1.27 ppm for H-1 and δ 32.94 ppm for C- 1. Correlation between δ 3.62 for H-26 ppm and δ 63.25 ppm for C-26.

### β-sitosterol

This compound was obtained as whitish crystal residue ( 20.8 mg) with R_{f} = 0.45 in benzene ethyl acetate 80:20. **¹H-NMR:** (Chloroform-D3) δ 5.34 ppm (1H d, H-7) (double bound) δ 3.51 ppm (1H, s, -OH), δ 2.26 ppm (2H q, H-3) nearest from -OH , δ 1.98 ppm (2H,t, H-5 & H-8), δ 1.83 ppm (3H, t, H-28), 1.63 ppm (1H, s , H-18), 1.57 ppm (8H, s, H-1, H-2, H-15 & H-16), 1.33 ppm (5H, m, H-9, H-11, & H-12), 1.14 ppm (6H, m, H-4, 24, 21, 17 & 22), 1.12 ppm (6H, d, H-29 & H-30), 0.91 ppm (4H d, H-19 & H-20), 0.81 ppm (9H, s, H-24, H-25 & H-26), 0.66 ppm (3H, s, H-23). **¹³C-NMR**: (CDCl₃) showed 30 carbons, and DEPT-135 revealed the presence of three quaternary carbon C-6 δ 140.87 ppm, δ 42.43 ppm (C-10), δ 36.62 ppm (C-13). δ 121.86 ppm (C-7), δ 71.93 ppm (C-3) -OH group, δ 56.87 ppm (C-17), δ 56.14 ppm (C-14), δ 50.23 ppm (C-21), δ 45.93 ppm (C-22), δ 42.42 ppm (C-19), δ 39.88 ppm (C-4), δ 37.36 ppm (C-12), δ 36.62 ppm (C-2), δ 36.27 ppm (C-18), δ 34.04 ppm (C-20), δ 32.03 ppm (C-1), δ 32.01 ppm (C-8), δ 31.78 ppm (C-15), δ 29.23 ppm (C-9), 28.38 ppm (C-16), δ 26.13 ppm (C-20), δ 24.43 ppm (C-), 22.08 ppm (C-24), δ 23.16 ppm (C-15), δ 21.20 ppm (C-27), δ 19.96 ppm (C-23), δ 19.53 ppm (C-24), δ 19.13 ppm (C-25), δ 18.90 ppm (C-29,C-30), δ 12.10 ppm (C-26), 11.98 ppm (C-28).

### kaempferol-3-O-β-D-glucoside

Yellow crystals (23 mg) R_{f} = 0.82 benzene ethyl acetate 80:20. UV (λ max) in MeOH : (nm) 265, 325(sh), 350; (AlCl₃): 265, 290 (sh), 420; (AlCl₃/ HCl) 265, 300, 420 (NaOAc) 215, 300, 365 (NaOAc/H₃BO₃) 270, 360 (NaOMe) 275, 325, 400 .¹H-NMR ( DMSO- d₆ ) : δ 7.9 ppm (2H, d, J = 9 Hz, H2 and H6); δ 6.7 ppm (2H, d, J = 9 Hz, H3 and H5); δ 5.6 ppm (1H, d, J = 2.5 Hz, H8); δ 5.5 ppm (1H, d, J = 2.5 Hz, H6); δ 5.1 ppm (d, J=7 Hz, H 1" glucose) and δ 3.1-3.8 ppm (m, remaining sugar protons). ¹³C NMR (DMSO) δ : 153.3 ppm (C-2) ; 132.6 ppm (C-3); 174.3 ppm (C-4); 159.6 ppm (C-5); 98.6 ppm (C-6); 160.4 ppm (C-7); 95.5 ppm (C-8);153.2 ppm (C-9); 103.4 ppm (C-10); 121.1 ppm (C-1'); 130.3 ppm (C-2'); 114.8 ppm (C-3'); 157.4 ppm (C-4'); 114.8 ppm (C-5'); 130.3 ppm (C-6'); 102.2 ppm (C-1"); 75.4 ppm (C-2"); 79.0 ppm (C-3"); 71.2 ppm (C-4"); 79.0 ppm (C-5"); 59.8 ppm(C-6"). EI-MS m/z (% re. Int.): 447 (M⁺) (100), 285 (95), 315 (18), 448 (68).

### kaempferol-3-galactoside

Yellow crystals (21mg) R_{f} = 0.40 benzene-ethyl acetate 80:20. UV (λ max) in MeOH : (nm) 265, 350; (AlCl₃)265, 300(sh), 410 (AlCl₃/ HCl) 265, 350, 410 (NaOAc) 275, 300, 380 (NaOAc/H₃BO₃) 270, 310, 375 (NaOMe) 275, 330, 400. ¹H-NMR (DMSO- d₆) : δ 7.9 ppm (2H, d, J = 8 Hz, H2 and H6), δ 6.8 ppm (2H, d, J = 8 Hz, H3 and H5), δ 5.8 ppm (1H, d, J = 2.5 Hz, H8), δ 5.6 ppm (1H, d, J = 2.5 Hz, H6), δ 5.4 ppm (1 H, d, anomeric proton),. ¹³C NMR (DMSO) : δ 153.2 ppm (C-2); 132.6ppm (C-3); 174.3 ppm (C-4); 159.6 ppm (C-5); 98.8 ppm (C-6); 160.4 ppm (C-7); 93.9 ppm (C-8); 153.2 ppm (C-9); 103.4 ppm (C-10); 121.1 ppm (C-1'); 130.3 ppm (C-2'); 114.8 ppm (C-3'); 157.4 ppm (C-4'); 114.7 ppm (C-5'); 130.3ppm (C-6'); 102.5 ppm (1"); 71.2 ppm (2"); 73.2 ppm (3"); 68.5 ppm (4"); 75.5 ppm (5"); 59.8 ppm (6").

### Example 2

### Pharmacological study

**Animals:** Swiss albino mice of both sex (26-30 g b.wt) and male Wistar albino rats (180-200 g b.wt) were used. Animals were maintained under standard conditions of temperature (23±1.0 °C), humidity (55±10%) and 12 h light/12 h dark cycle and fed with a standard pellet diet with water *ad libitum*. All the rats were housed in standard polypropylene cages with wire mesh top. Animals were allowed to adapt to the laboratory environment for one week before experimentation.

**Preparation of the extracts for biological studies:** The total alcohol extract of *Euphorbia granulata* Forrsk. (6. granulate) and the standard drug (dexamethasone) were suspended separately in 3% v/v Tween 80 (vehicle).

The total alcohol extract was obtained by direct extraction of *Euphorbia granulate* Forrsk. with ethanol. The extraction conditions were chosen as mentioned above.

**Acute toxicity (LD₅₀) test:** The oral median lethal doses (LD₅₀) of the alcohol extract of *E. granulata* was determined as described by Lorke (1983). Swiss albino mice, in groups of six, received one of 1000, 2000 or 4000 mg/kg of the tested extract suspended in the vehicle. Control animals received the vehicle and were kept under the same conditions. Signs of acute toxicity and number of deaths per dose within 24 h were recorded and the LD₅₀ was calculated as the geometric mean of the dose that resulted in 100% mortality and that which caused no lethality at all.

***Doses:*** In this investigation, an experimental dose of 400 mg/kg that equals to ¹/₁₀ of the maximum possible dose of the alcohol extract that didn't cause mortalities in mice was selected to be given orally to rats. The reference drug, dexamethasone, was given orally at dose of 0.1 mg/kg. These doses were calculated by converting the therapeutic doses that used in human to rat's doses according to the Table of Paget and Barnes (1964).

**Sub-chronic Toxicity:** Wistar albino rats were randomly divided into 3 equal groups. Rats of the 1^{st} group were given the vehicle in a dose of 5 mL/kg and left as normal control. Rats of the 2^{nd} group were administered the alcohol extract of *E. granulata,* in a dose of 400 mg/kg, while the 3^{rd} group received dexamethasone. All medications were administered orally via the aid of an orogastric cannula for 35 consecutive days. Animals were maintained under identical conditions with food and water *ad libitum* for the entire period with close observation. At the end of the experimental period, blood samples (2 mL) were drawn by puncturing retro-orbital venous sinus of each rat (under ether anesthesia) and centrifuged at 10,000 rpm for 5 minutes. Sera were separated to be used for the biochemical estimations.

**Measurement of liver and kidney function markers:** Liver functions were evaluated by measuring the serum activity of alanine aminotransferase (ALT) and aspartate aminotransferase (AST) following the method of Reitman and Frankel (1957). Serum levels of total bilirubin (Walter and Gerarade, 1970), total proteins (Henary *et al.,* 1974) and albumin (Doumas *et al.,* 1971) were also assayed. Serum concentrations of urea (Wills and Savory, 1981) and creatinine (Kroll *et al*., 1987) were determined colormetrically as measures of kidney functions.

**Effect on ulcerative colitis:** Male Wistar albino rats were divided into 9 experimental groups, each of 6 animals. Rats of groups 1 and 2 (Sham and Control colitis groups, respectively) were given the vehicle only in a dose of 5 mL/kg. Group 3 (Reference group) were given dexamethasone in a dose of 0.1 mg/kg. Rats of the 4^{th} group received the alcohol extract of *E. granulata* in a dose of 400 mg/kg. Groups from 5^{th} to 9^{th} received the isolated compounds at a dose of 100 mg/kg. All medications were administered orally, once daily via the aid of an orogastric cannula for 5 successive days and the last dose was administered 2 h before colitis induction.

**Induction of ulcerative colitis:** Before induction of the ulcerative colitis, all animals were fasted overnight, with access to water *ad libitum*, and then anesthetized with ether inhalation. A polyethylene catheter with 2 mm diameter was inserted into the lumen of the colon via the anus to a distance of 8 cm (Mascolo *et al*., 1995). A solution of 2 mL (4%, v/v) acetic acid in saline was slowly infused into the colon through the catheter. The rats were then maintained in a supine Trendelenburg position for 30 seconds to prevent early leakage of the acetic acid (Millar *et al*., 1996). In sham group, equivolume of normal saline was infused into the colon instead of acetic acid. The acetic acid was then aspirated and 2 mL of phosphate buffer solution (pH=7) was instilled into the rectum of each rat (Garg *et al*., 1991). The catheter was left in place for a few seconds then gently removed. Two days after the induction of colitis, each animal was sacrificed using ether anesthesia and laparatomy was performed. Colonic segments (8 cm in length and 3 cm proximal to the anus) were excised, opened along its mesenteric border, washed with saline and were used for macroscopic scoring.

**Assessment of colonic lesions:** The colon specimens were weighted and wet weight/length ratio was calculated for all the rats. The specimens were examined under a dissecting microscope and the mucosal lesions were quantified by the scoring system (0-5) given by Millar *et al*., (1996) after some modifications.

*The lesion scores* were: 0 = no damage, 1 = Local edema and inflammation without ulcers; 2 = One ulcer without inflammation; 3 = one to two ulcers with inflammation & lesion diameter < 1 cm; 4 = More than two ulcers with lesion diameter 1-2 cm; 5 = Sever ulceration with lesion diameter >2 cm.

*Ulcer area* was measured using plane glass square. Each cell on the glass square was 1 mm² in area and the number of cells was counted and the ulcer area was determined for each colon. *Ulcer index* was measured by summing the lesion score and the ulcer area for each colon specimen (Minaiyan *et al*, 2006).

**Statistical Analysis:** Data were expressed as mean ± S. E. M. Statistical analysis was done by using SPSS 10. Statistical significance of differences between two means was assessed by unpaired Student's 't' test. Differences at *p* < 0.05, 0.01, and 0.001 were considered statistically significant.

### Results

### Acute toxicity (LD₅₀) test:

The tested extract is characterized by a low degree of toxicity. The obtained results indicated that different doses of the alcohol extract *E. granulata* (1000, 2000 and 4000 mg/kg) did not produce any symptom of acute toxicity and none of the mice died during 24 h of observation.

All mice did not exhibit diarrhoea, haematuria, restlessness, uncoordinated muscle movements, and respiratory distress. Accordingly, it is suggested that the oral LD₅₀ of the tested extracts is higher than 4000 mg/kg. Accordingly, the tested extracts are considered to be safe since substances possessing LD₅₀ higher than 50 mg/kg are non toxic (Buck *et al.,* 1976).

### Sub-chronic toxicity:

The non toxic nature of the alcohol extract of *E. granulata* in the acute toxicity study is well supported by the biochemical data following 35-days treatment period in rats. In the present study, oral dosing of the tested extracts to rats in a dose of 400 mg/kg for 35 days did not show any significant effect on the levels of ALT, AST, total bilirubin, total proteins and albumin in their sera as compared to control (Table 1). The serum transaminase level is most widely used as a measure of hepatic injury due to its ease of measurement and high degree of sensitivity. It is useful for the detection of early damage of hepatic tissue. Since the activity of ALT and AST are specific assayable liver enzymes, their normal levels in serum of experimental groups of rats treated for 35 days means that the alcohol extract of *E. granulata* is not hepatotoxic.

The tested extract showed significant change in the mean values of urea and creatinine in sera of rats following 35 days of administration at 400 mg/kg dose when compared with the control rats (Table 2). Urea and creatinine are the most sensitive biochemical markers employed in the diagnosis of renal damage. In kidney damage, there will be retention of urea and creatinine in the blood. Therefore, marked increase in serum urea and creatinine are indications of functional damage to the kidney. By these indicators, the alcohol extract of *E. granulata* is not nephrotoxic in rats.

### Effect on ulcerative colitis:

The model of acetic acid induced colitis shares many of the histologic features of ulcerative colitis in human beings including mucosal edema and submucosal ulceration. In rats of sham group, no abnormal changes were observed suggesting that the handling procedure had no interference with the experimental outputs. Macroscopic damage parameters of the colon of control colitis rats, 2 days after rectal infusion of acetic acid revealed dark brown lesions, mucosal hyperemia, edema, erosion, and ulceration. Control colitis rats showed lesion score, ulcer area and ulcer index values of 4.7±0.29, 5.5±0.34 cm² and 10.2±0.63, respectively (Table 3). The inflammatory changes of the intestinal tract were associated with a significant increase of wet weight/length of the colon specimens as an indicator of inflammation. These inflammatory indices were significantly improved by oral dosing of dexamethasone (P<0.001), the alcohol extract of *E. granulata* and the isolated compounds for 5 days prior to ulcer induction. The anti-ulcerative effect of the extract was lower than that of dexamethasone while all the isolated compounds were more effective than dexamethasone.

**Table 1: Effect of prolonged oral administration of the tested plant extracts in a dose of 400 mg/kg for 35 consecutive days on the serum activity of ALT and AST and serum levels of total bilirubin, total protein, and albumin in rats, (n=6).**

| **Groups** | **ALT (U/L)** | **AST (U/L)** | **T. bilirubin (mg/dL)** | **T. protein (g/dL)** | **Albumin (g/dL)** |
|---|---|---|---|---|---|
| Control | 67.5±3.48 | 146.3±5.20 | 1.7±0.11 | 8.6±0.22 | 3.7±0.01 |
| *E. granulata* | 69.3±2.73 | 160.4±6.54 | 1.7±0.10 | 8.3±0.34 | 3.2±0.25 |

**Table 2: Effect of prolonged oral administration of the tested plant extract in a dose of 400 mg/kg for 35 consecutive days on the serum levels of urea and creatinine in rats, (n=6).**

| Groups | **Urea (mg/dL)** | **Creatinine (mg/dL)** |
|---|---|---|
| Control | 37±2.35 | 0.43±0.22 |
| *E. granulata* | 38±2.55 | 0.40±0.25 |

**Table 3: Effects of dexamethasone (0.1 mg/kg), the tested extract (400 mg/kg) and isolated compounds (100 mg/kg) on the macroscopic parameters of ulcerative colitis induced by acetic acid in rats. (n = 6).**

| Groups | **Lesion score (0-5)** | **Ulcer area** (**cm²**) | **Ulcer index** | **Wet W/L (g/cm)** |
|---|---|---|---|---|
| Sham | 0.0 ± 0.0 | 0.0 ± 0.0 | 0.0 ± 0.0 | 0.34 ± 0.03 |
| Control colitis | 4.7 ± 0.29 | 5.5 ± 0.34 | 10.2 ± 0.63 | 0.98 ± 0.07 |
| Dexamethasone | 2.0 ± 0.12*** | 2.4 ± 0.15*** | 4.4 ± 0.28*** | 0.39 ± 0.02*** |
| *E. granulata* | 3.3 ± 0.24** | 3.6 ± 0.27** | 6.9 ± 0.39** | 0.55 ± 0.04*** |
| heptacosan-1-ol | 1.3 ± 0.19*** | 1.6 ± 0.25*** | 2.9 ± 0.27*** | 0.48 ± 0.04*** |
| β-sitosterol | 1.5 ± 0.20*** | 2.0 ± 0.28*** | 3.5 ± 0.31*** | 0.53 ± 0.06*** |
| kampferol-3-glucoside | 1.7 ± 0.22*** | 2.2 ± 0.19*** | 2.9 ± 0.28*** | 0.55 ± 0.03*** |
| kampferol-3-glucoside | 1.5 ± 0.22*** | 2.0 ± 0.19*** | 2.5 ± 0.28*** | 0.51 ± 0.03*** |

| | | | | |
|---|---|---|---|---|
| *, **, *** Significant at p≤0.05 and p ≤0.01 and P ≤0.001 respectively | | | | |

The features disclosed in the foregoing description and claims may both separately and in any combination be material for realizing the invention in diverse forms thereof.

## Claims

1. Extract from a plant material of *Euphorbia granulata* Forssk. for use in the treatment of ulcerative colitis.

2. Extract for use according to claim 1, wherein the extract is an alcoholic extract, preferably is an ethanolic extract.

3. Extract for use according to claim 1 or 2, wherein the plant material comprises the aerial parts of *Euphorbia granulata* Forssk.

4. Compound selected from heptacosan-1-ol, kaempferol-3-glycoside and/or kaempferol-3-galactoside for use in the treatment of ulcerative colitis.

5. Method for isolating heptacosan-1-ol and/or kaempferol-3-galactoside from *Euphorbia granulata* Forssk., comprising the steps:
a) extracting a plant material of *Euphorbia granulata* Forssk. to obtain an extract;
b) concentrating the extract to obtain a concentrate;
c) chromatographing the concentrate into fractions; and
d) isolating fractionated compounds.

6. Method according to claim 5, wherein the plant material comprises the aerial parts of *Euphorbia granulata* Forssk.

7. Method according to claims 5 or 6, wherein extracting is carried out by using at least one organic solvent.

8. Method according to claim 7, wherein the organic solvent is selected from the group consisting of petroleum ether, heptane, hexane, ethanol, isopropanol, methanol and mixtures thereof, preferably from the group consisting of petroleum ether and ethanol.

9. Method according to any of the claims 5 to 8, wherein chromatographing is carried out by means of thin layer chromatography (TLC) and/or column chromatography.

10. Method according to any of the claims 5 to 9, wherein the eluent used for chromatographing is petroleum ether, heptane, ethanol, methanol, benzene, diethyl ether, chloroform, dichloromethane, water and/or mixtures thereof.

11. Method according to any of the claims 5 to 10, wherein the stationary phase used for chromatographing is silica gel or aluminum oxide.

12. Pharmaceutical composition comprising an extract according to claim 1 and/or at least one of the compounds according to claim 4 for use in the treatment of ulcerative colitis.

13. Pharmaceutical composition for use according to claim 12, formulated for oral administration.

## Patentansprüche

1. Extrakt eines Pflanzenmaterials von *Euphorbia granulata* Forssk. zur Verwendung in der Behandlung von Colitis ulcerosa.

2. Extrakt zur Verwendung nach Anspruch 1, wobei das Extrakt ein alkoholisches Extrakt ist, vorzugsweise ein ethanolisches Extrakt.

3. Extrakt zur Verwendung nach Anspruch 1 oder 2, wobei das Pflanzenmaterial die oberirdischen Teile von *Euphorbia granulata* Forssk. umfasst.

4. Verbindung ausgewählt aus Heptacosan-1-ol, Kaempferol-3-glycosid und/oder Kaempferol-3-galactosid zur Verwendung in der Behandlung von Colitis ulcerosa.

5. Verfahren zur Isolierung von Heptacosan-1-ol und/oder Kaempferol-3-galactosid aus *Euphorbia granulata* Forssk., umfassend die Schritte:
a) Extrahieren eines Pflanzenmaterials von *Euphorbia granulata* Forssk., um ein Extrakt zu erhalten;
b) Aufkonzentrieren des Extrakts, um ein Konzentrat zu erhalten;
c) Chromatographieren des Konzentrates in Fraktionen; und
d) Isolieren der fraktionierten Verbindungen.

6. Verfahren nach Anspruch 5, wobei das Pflanzenmaterial die oberirdischen Teile von *Euphorbia granulata* Forssk. umfasst.

7. Verfahren nach Anspruch 5 oder 6, wobei das Extrahieren unter Verwendung von zumindest einem organischen Lösungsmittel durchgeführt wird.

8. Verfahren nach Anspruch 7, wobei das organische Lösungsmittel ausgewählt ist aus der Gruppe, bestehend aus Petrolether, Heptan, Hexan, Ethanol, Isopropanol, Methanol und Gemischen derselben, vorzugsweise aus der Gruppe, bestehend aus Petrolether und Ethanol.

9. Verfahren nach einem der Ansprüche 5 bis 8, wobei das Chromatographieren mittels Dünnschichtchromatographie (TLC) und/oder Säulenchromatographie durchgeführt wird.

10. Verfahren nach einem der Ansprüche 5 bis 9, wobei das Eluent, das zum Chromatographieren verwendet wird, Petrolether, Heptan, Ethanol, Methanol, Benzol, Diethylether, Chloroform, Dichlormethan, Wasser und/oder Mischungen derselben, ist/sind.

11. Verfahren nach einem der Ansprüche 5 bis 10, wobei die stationäre Phase, die zum Chromatographieren verwendet wird, Kieselgel oder Aluminiumoxid ist.

12. Pharmazeutische Zusammensetzung, umfassend ein Extrakt nach Anspruch 1 und/oder zumindest eine der Verbindungen nach Anspruch 4 zur Verwendung in der Behandlung von Colitis ulcerosa.

13. Pharmazeutische Zusammensetzung zur Verwendung nach Anspruch 12, hergerichtet zur oralen Verabreichung.

## Revendications

1. Extrait d'un matériau végétal d*'Euphorbia granulata* Forssk. pour une utilisation dans le traitement de la rectocolite hémorragique.

2. Extrait pour une utilisation selon la revendication 1, l'extrait étant un extrait alcoolique et préférablement un extrait éthanolique.

3. Extrait pour une utilisation selon les revendications 1 ou 2, dans lequel le matériau végétal comprend les parties aériennes d'*Euphorbia granulata* Forssk.

4. Composé sélectionné parmi l'heptacosan-1-ol, le kaemptérol-3-glucoside et/ou le kaempférol-3-galactoside pour une utilisation dans le traitement de la rectocolite hémorragique.

5. Procédé d'isolement de l'heptacosan-1-ol et/ou du kaempférol-3-galactoside à partir d*'Euphorbia granulata* Forssk., qui comprend les étapes consistant à :
a) extraire un matériau végétal d'*Euphorbia granulata* Forssk. pour obtenir un extrait ;
b) concentrer l'extrait pour obtenir un concentré ;
c) chromatographier le concentré en fractions; et
d) isoler les composés fractionnés.

6. Procédé selon la revendication 5, dans lequel le matériau végétal comprend les parties aériennes d'*Euphorbia granulata* Forssk.

7. Procédé selon les revendications 5 ou 6, dans lequel l'extraction est effectuée en utilisant au moins un solvant organique.

8. Procédé selon la revendication 7, dans lequel le solvant organique est sélectionné dans le groupe consistant en l'éther de pétrole, l'heptane, l'hexane, l'éthanol, l'isopropanol, le méthanol et des mélanges de ceux-ci, préférablement dans le groupe consistant en l'éther de pétrole et l'éthanol.

9. Procédé selon l'une quelconque des revendications 5 à 8, dans lequel la chromatographie est effectuée par chromatographie sur couche mince (CCM) et/ou par chromatographie sur colonne.

10. Procédé selon l'une quelconque des revendications 5 à 9, dans lequel l'éluant utilisé pour effectuer la chromatographie est l'éther de pétrole, l'heptane, l'éthanol, le methanol, le benzène, l'éther diéthylique, le chloroforme, le dichlorométhane, l'eau et/ou des mélanges de ceux-ci.

11. Procédé selon l'une quelconque des revendications 5 à 10, dans lequel la phase stationnaire utilisée pour effectuer la chromatographie est un gel de silice ou l'oxyde d'aluminium.

12. Composition pharmaceutique comprenant un extrait selon la revendication 1 et/ou au moins un des composés selon la revendication 4 pour une utilisation dans le traitement de la rectocolite hémorragique.

13. Composition pharmaceutique pour une utilisation selon la revendication 12, formulée pour être administrée par voie orale.
